# EUROPEAN PATENT SPECIFICATION

(11) **EP 1 890 621 B1**
(45) Date of publication and mention of the grant of the patent: **18.09.2013**
(21) Application number: 06739809.9
(22) Date of filing: 28.03.2006
(51) Int. Cl.: A61B 17/70

(54) **SPINAL ROD CONNECTOR**
WIRBELSÄULEN-VERBINDUNGSSTANGE
CONNECTEUR DE BROCHE SPINALE

(30) Priority: 30.03.2005 US 93487
(43) Date of publication of application: 27.02.2008
(73) Proprietor: Warsaw Orthopedic, Inc., Warsaw, IN 46581 (US)
(72) Inventor: AVERY, Syeven, J., Arlington, Tennessee 38002 (US); FARRIS, Robert, A., Cordova, Tennessee 38018 (US); MAY, Jason M., Cordova, TN 38016 (US)
(74) Representative: Hutchinson, Glenn Stanley
(86) International application number: PCT/US2006/011245
(87) International publication number: WO 2006/105069

(56) References cited:
- EP-A1- 0 469 304
- EP-A1- 1 260 187
- WO-A-02/24087
- WO-A-2005/102189
- WO-A-2005/122922
- WO-A-2006/001993
- WO-A2-02/34150
- JP-A- 11 244 299

## Description

### BACKGROUND

The spine is subject to various pathologies that compromise its load bearing and support capabilities. Such pathologies of the spine include, for example, degenerative diseases, the effects of tumors and, of course, fractures and dislocations attributable to physical trauma. In the treatment of diseases, malformations or injuries affecting spinal motion segments (which include two or more adjacent vertebrae and the disc tissue or disc space therebetween), and especially those affecting disc tissue, it has long been known to remove some or all of a degenerated, ruptured or otherwise failing disc. It is also known that artificial discs, fusion implants, or other interbody devices can be placed into the disc space after disc material removal. External stabilization of spinal segments alone or in combination with interbody devices also provides advantages. Elongated rigid plates, rods and other external stabilization devices have been helpful in the stabilization and fixation of a spinal motion segment, in correcting abnormal curvatures and alignments of the spinal column, and for treatment of other conditions.

In spinal rod fixation procedures extending to the base of the skull, the surgeon contours the rod to match the angle of the occiput and sub-axial spine. Bending the rod induces stress to the rod and decreases the fatigue strength of the material. In addition, the geometric and dimensional features of these rod systems and patient anatomy constrain the surgeon during surgery and prevent optimal placement and attachment along the spinal column and the occiput. For example, elongated, one-piece rods can be difficult to bend and maneuver into position between the upper end of the cervical spine and the occipital bone of the skull, and also provide the surgeon with only limited options in sizing and selection of the rod system to be placed during surgery.

The document EP-A-1260187 describes a device for external transpedicular vertebral column fixation that is used for curing spinal diseases and injuries in specialized departments of vertebrology and neurosurgery. The device comprises not more than nine fixing units where each unit bears two fixing elements fastened to it which are embodied in the form of a threaded stem, the working end of which are implanted in roots of vertebra arches angularly at 35-45 DEG to the sagittal plane of the vertebra. The fixing units are connected to each other in groups of three with the aid of threaded ties, thereby forming support units. Two of the units are side units and one is an intermediate unit. The intermediate unit is connected to the side support units so that it can move with the aid of the connecting hinge junctions. Dosed displacement is performed by means of moving distraction nuts. Displacement of noninvolved vertebrae is carried out until a normal correction of the spinal column occurs and ostial and ostial -fibrotic blocks are built up in the area of discotomy of vertebrae Th8 and Th9.

### SUMMARY OF THE INVENTION

The present invention is defined in the appended claims.

In one aspect, a spinal rod connection system having a first elongated rod for attachment to the spinal column. The first rod extends along a longitudinal axis between a coupling member at its first end and an opposite second end. The system further includes a second elongated rod for attachment to a skull. Similarly, the second rod also extends along a longitudinal axis between a coupling member at its first end and an opposite second end. The system further includes a coupling mechanism for releasably coupling the coupling member of the first rod and the coupling member of the second rod in an end-to-end fashion where the longitudinal axis of each of the first and second rods is at an angle to one another.

In another aspect, a spinal rod connection system having a first elongated rod for attachment to the spinal column. The first rod extends along a longitudinal axis between a coupling member at its first end and an opposite second end. The system further includes a second elongated rod for attachment to the skull. Similarly, the second rod also extends along a longitudinal axis between a coupling member at its first end and an opposite second end. The system further includes a coupling mechanism for releasably coupling the coupling member of the first rod and the coupling member of the second rod in an end-to-end fashion, wherein an angle between the longitudinal axis of each of the rods can be temporarily varied and thereafter fixed so that the coupling mechanism axially and torsionally constrains the rods relative to one another.

Further disclosed is a non-claimed method for assembling a spinal rod system extending to the skull. The method includes a first rod that extends along a longitudinal axis between a coupling member at its first end and an opposite second end. The method further includes a second elongated rod for attachment to the skull. Similarly, the second rod also extends along a longitudinal axis between a coupling member at its first end and an opposite second end. The method further includes providing a coupling mechanism for releasably coupling said first coupling member and said second coupling member in an end-to-end fashion, wherein said longitudinal axis of each of said first and second rods is at an angle to one another. The method may further include temporarily varying the angle and thereafter fixing it so that the coupling mechanism axially and torsionally constrains the first and second rods relative to one another.

### BRIEF DESCRIPTION OF THE FIGURES

Fig. 1 is an exploded perspective view of one embodiment of the spinal rod connection system.
Fig. 2 is a side view of the spinal rod connection system of Fig. 1 assembled.
Fig. 3 is an exploded perspective view of another embodiment of the spinal rod connection system.
Fig. 4 is a side view of the spinal rod connection system of Fig. 3 assembled.
Fig. 5 is an exploded perspective view of another embodiment of the spinal rod connection system.
Fig. 6 is a side view of the spinal rod connection system of Fig. 5 assembled.
Fig. 7 is an exploded perspective view of another embodiment of the spinal rod connection system.
Fig. 8 is a side view of the spinal rod connection system of Fig. 7 assembled.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is hereby intended. Any such alterations and further modifications in the illustrated devices, and any such further applications of the principles of the invention as illustrated herein being contemplated as would normally occur to one skilled in the art to which the invention relates.

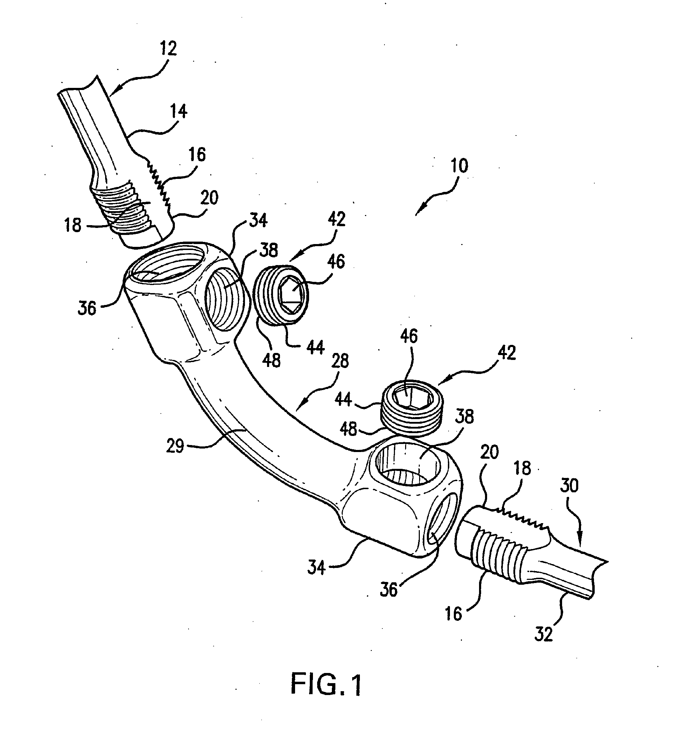

In Fig. 1 there is shown a spinal rod connection system 10 including a first rod 12 and a second rod 30. First rod 12 and second rod 30 are releasably coupled to one another in end-to-end fashion with a coupling mechanism 28. The coupling mechanism 28 is configured to secure rods 12, 30 to one another in end-to-end fashion while providing the necessary angulation between the axis of the rods to conform to the angle of the occiput and the sub-axial spine. This minimizes the footprint or intrusiveness of the coupling mechanism into the tissue surrounding the rod system, and decreases or potentially eliminates, the amount that each rod is contoured to conform to the angle of the occiput and the sub-axial spine.

First rod 12 includes a rod portion 14 and coupling member 16 at one end of rod portion 14. Second rod 30 includes a rod portion 32 and coupling member 16 at one end of rod portion 32. Coupling mechanism 28 includes a rod portion 29 and a coupling body 34 at either end thereof. Rod portion 29 may be of any shape and can be configured in any number of angles or configurations. Rod portions 14, 32 can be secured to vertebrae of the spinal column system or to the occiput of the skull with any one or combination of plates, hooks, screws, bolts, multi-axial screws, staples, cables or wires, sutures, clamps, and/or other attachment devices and systems.

First rod portion 14 can be provided with a characteristic that differs from a characteristic of second rod portion 32. The coupling mechanism 28 allows rods of differing characteristics to be secured to one another in end-to-end fashion to provide a rod system that is adapted for the anatomy, surgical condition, or surgical procedure. In one embodiment, the characteristic includes a cross-sectional dimension of the rod portions 14, 32. Other embodiments contemplate selection criteria for selection and assembly of the rod portion to include any one or combination of characteristics, including length, contouring, flexibility, surface features, shape, section modulus, elasticity, materials and material properties, and coatings, for example.

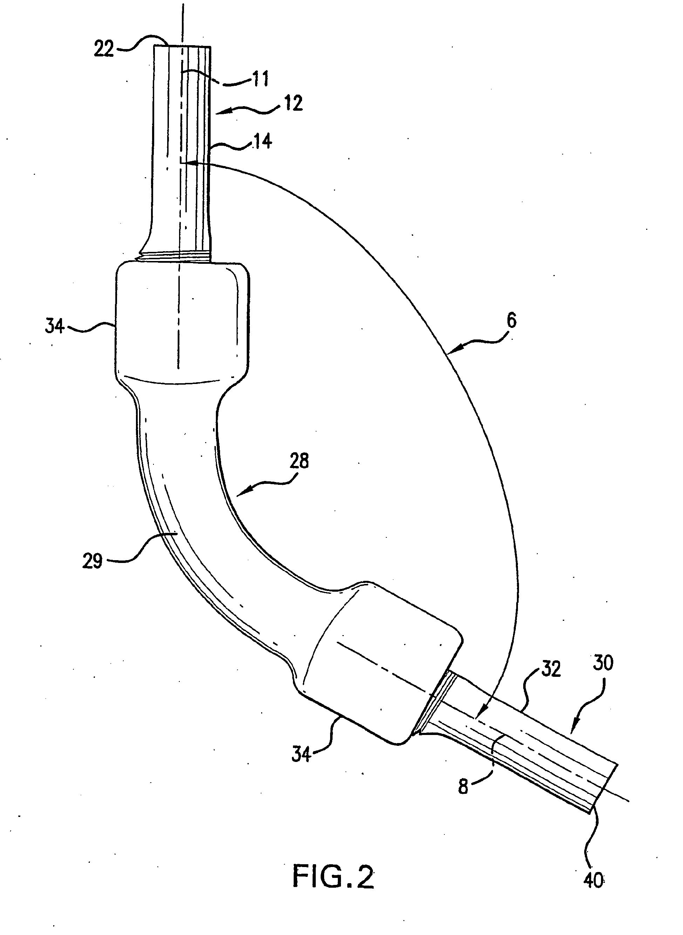

As shown in Figs. 1-2, rod portion 14 extends along longitudinal axis 11 and includes a first cross-sectional dimension 22 between opposite sides thereof transverse to longitudinal axis 11. Similarly, rod portion 32 extends along longitudinal axis 8 and includes a second cross-sectional dimension 40 between opposite sides thereof transverse to longitudinal axis 8. Cross-sectional dimension 22 corresponds to a diameter of a cylindrical rod portion 14 that may be smaller, larger or the same as the diameter corresponding to cross-sectional dimension 40 of a cylindrical rod portion 32. In one specific application, the diameter of first rod portion 14 is sized to extend along a first portion of the spine, such as the cervical region, and the diameter of second rod portion 32 is sized to extend up to the skull region. As shown in Fig. 2, axis 11 is at an angle 6 from axis 8.

Coupling member 16 includes a threaded outer surface extending from rod portion 14 or 32 to an end member 20 lacking threads. Coupling member 16 further includes a contact portion 18 along at least one side thereof, as also shown in Fig. 1. According to the invention, the contact portion is a flattered surface portion interrupting an external thread pattern included in the first and second coupling members. Contact portion 18 can be formed by a cutout providing a flat surface extending along coupling member 16. Contact portion 18 can also include a concave surface, a convex surface, a receptacle, or other suitable configuration for contacting an engaging member. The thread pattern of coupling member 16 extends completely therearound between the opposite sides of contact portion 18. Other embodiments contemplate that multiple contact portions are provided along coupling member 16 to provide multiple engagement locations for engaging member 42, as discussed further below.

Coupling body 34 can be enlarged relative to rod portion 29 to provide a flange or hub to which coupling member 16 can be releasably engaged. In the illustrated embodiment, coupling body 34 is a cubic block, although other shapes are also contemplated, such as rectangular and cylindrical shapes. Coupling body 34 includes a first bore 36 formed internally therein that opens away from rod portion 29 at an end of coupling body 34. Coupling body 34 further includes a second bore 38 extending therein transversely to first bore 36. As further shown in Fig. 1, second bore 38 can be internally threaded for receipt of an engagement member. Second bore 38 can also be orthogonal to first bore 36, although other orientations are also contemplated.

As shown in Fig. 2, rods 12, 30 can be assembled to coupling mechanism 28 whereby coupling members 16 are received in bores 36 and axially constrained by threaded engagement with coupling bodies 34. End members 20 facilitates positioning and alignment of coupling members 16 in bores 36, preventing or reducing the possibility of cross-threading coupling members 16 with coupling bodies 34. The axial load between rods 12, 30 is carried by the engagement of the coupling mechanism 28 to rods 12, 30. Accordingly, torsional loading of the components of rod system 10 is minimized since the rods 12, 30 are connected to one another by the coupling mechanism 28 in end-to-end fashion. Furthermore, coupling mechanism 28 may be shaped such that when rod system 10 is assembled, axis 11 of rod 12 is at an angle 6 from axis 8 of rod 30, as seen in Fig. 2. Angle 6 may match the angle of the occiput and sub-axial.

Engaging member 42 is positionable in second bore 38 and engageable to coupling member 16 to prevent rod 12 or 30 from disengaging from coupling mechanism 28. In one embodiment, contact portion 18 of coupling member 16 is oriented toward second bore 38, and engaging member 42 is an externally threaded set screw 44 engageable in second bore 38. A tool engaging recess 46 is provided for engagement with a tool, such as a hex driver, to allow the set screw 44 to be driven into second bore 38. Set screw 44 is driven into second bore 38 so that end 48 is in contact with contact portion 18, preventing coupling member 16 from rotating in coupling body 34 and resisting torsional loading between rods 12, 30.

Other embodiments contemplate other arrangements for coupling mechanism 28. For example, engaging member 42 can be a pin that is received in a hole or recess in coupling member 16. Coupling body 34 can be a nut rotatably captured on the end of rod portion 32. Coupling body 34 can include a bayonet locking type device, or a spring-biased ball plunger in coupling member 16 that is received in a detent in coupling body 34. In another non-claimed example, coupling body 34 does not include a second bore for an engaging member, and coupling member 16 can be provided completely threaded therearound. To torsionally constrain this type of coupling member 16 in coupling body 34, coupling member 16 can be provided with locking threads. In another example, a simple threaded engagement is provided and relative rotation of rods 12, 30 is resisted by the engagement of rods 12, 30 to the spinal column or the skull.

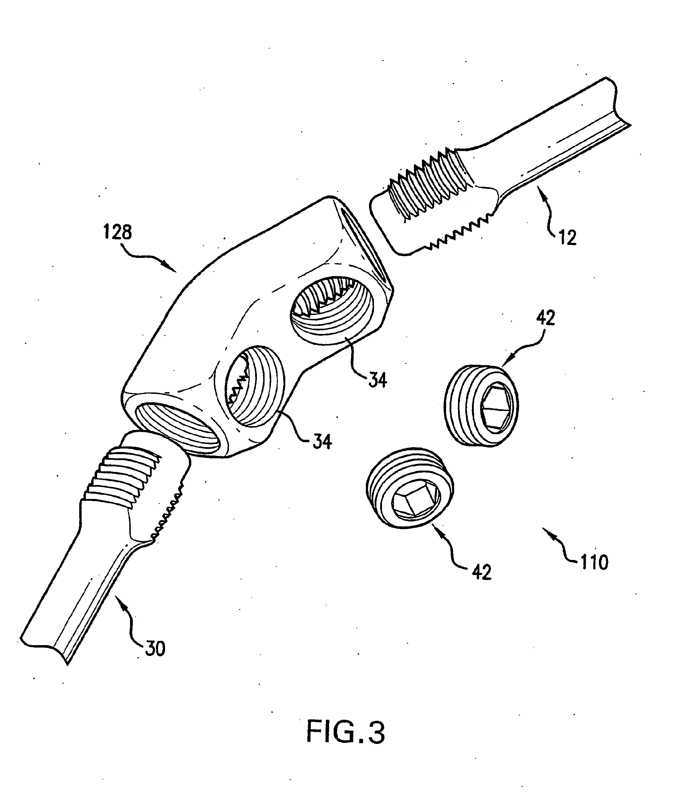

In Figs. 3-4, there is shown another embodiment of the spinal rod connection system 110. Many elements of this embodiment are identical to the elements found in the above embodiment and will not be repeated here. First rod 12 and second rod 30 are releasably coupled to one another in end-to-end fashion with a coupling mechanism 128. The coupling mechanism 128 is configured to secure rods 12, 30 to one another in end-to-end fashion while providing the necessary angulation between the axis of the rods to conform to the angle of the occiput and the sub-axial spine. In this embodiment, coupling mechanism 128 comprises coupling bodies 34 that are directly connected to one another. This embodiment further minimizes the footprint or intrusiveness of the coupling mechanism into the tissue surrounding the rod system while providing all of the same features of the embodiments described above.

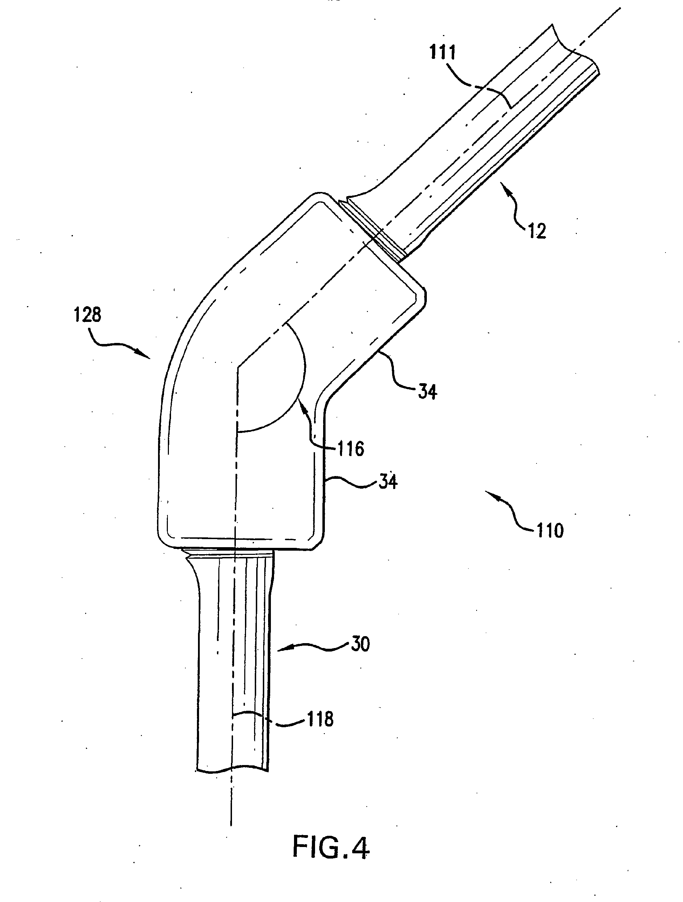

As shown in Fig. 4, coupling mechanism 128 may be shaped such that when rod system 110 is assembled, axis 111 of rod 12 is at an angle 116 from axis 118 of rod 30. Angle 116 may match the angle of the occiput and sub-axial spine.

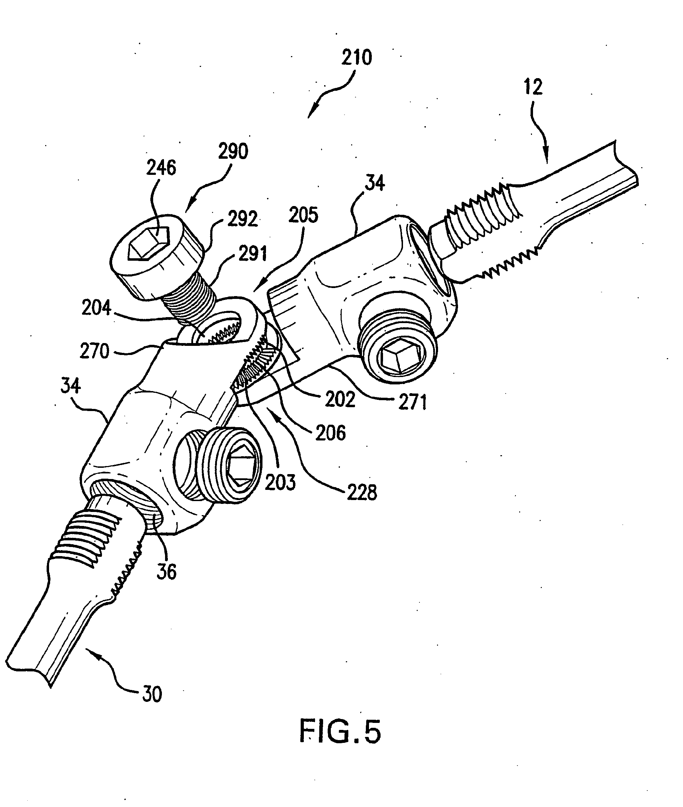

In Figs. 5-6, there is shown another embodiment of the spinal rod connection system 210. Many elements of this embodiment are identical to the elements found in the above embodiments and will not be repeated here. First rod 12 and second rod 30 are releasably coupled to one another in end-to-end fashion with a coupling mechanism 228. The coupling mechanism 228 is configured to secure rods 12, 30 to one another in end-to-end fashion while providing the necessary angulation between the axis of the rods to conform to the angle of the occiput and the sub-axial spine. In this embodiment, coupling mechanism 228 comprises an articulating connector 205 and coupling bodies 34 at either end thereof. This embodiment includes a articulating connector 205 which allows a surgeon to set and then fix the necessary angulation between the axis of the rods to conform to the angle of the occiput and the sub-axial spine while providing all of the same features and of the embodiments described above.

In Fig. 5, there is shown articulating connector 205 comprising extension portions 270, 271 which are respectively located at the end of coupling bodies 34 opposite of first bores 36. Extension portion 270 has a contact surface 202 that is planar and parallel to axis 218. Extension portion 271 has a contact surface 203 that is planar and parallel to axis 211.

Articulating connector 205 further includes a fastener 290 extendable through aligned bores 204, 206. Aligned bore 204 extends through extension portion 270 and its axis is perpendicular to contact surface 202. Aligned bore 206 extends through extension portion 271 and its axis is perpendicular to contact surface 203.

Fastener 290 is positionable through bore 204 and is engageable to bore 206 of extension portion 271 to prevent contact surfaces 202 and 203 from moving relative to one another. In one embodiment, fastener 290 has an externally threaded portion 291 engageable in bore 206, which has internal threads. A tool engaging recess 246 is provided for engagement with a tool, such as a hex driver, to allow the fastener 290 to be driven into bore 206. Fastener 290 is driven into bore 206 so that end 292 is in contact with extension portion 270 and contact surfaces 202 and 203 are in contact with one another and are prevented from moving relative to one another and resisting torsional loading between coupling bodies 34. One or both of contact surfaces 202, 203 may include teeth, splines, cams, roughened portions or any other movement prevention device to help prevent the contact surfaces from moving relative to one another.

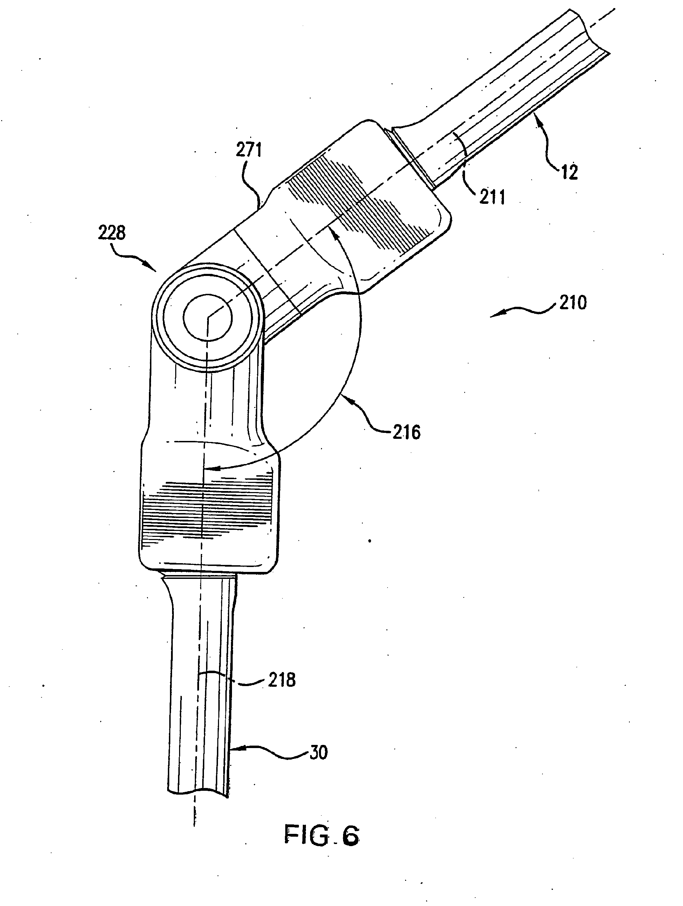

As shown in Fig. 6, spinal rod connection system 210 may be may be assembled so that axis 211 of rod 12 is at an angle 216 from axis 218 of rod 30. Prior to tightening fastener 290, angle 216 may be selected to match the angle of the occiput and sub-axial spine. Even after fastener 290 is tightened, it may be loosened so that another angle 216 can be selected and fastener 290 re-tightened.

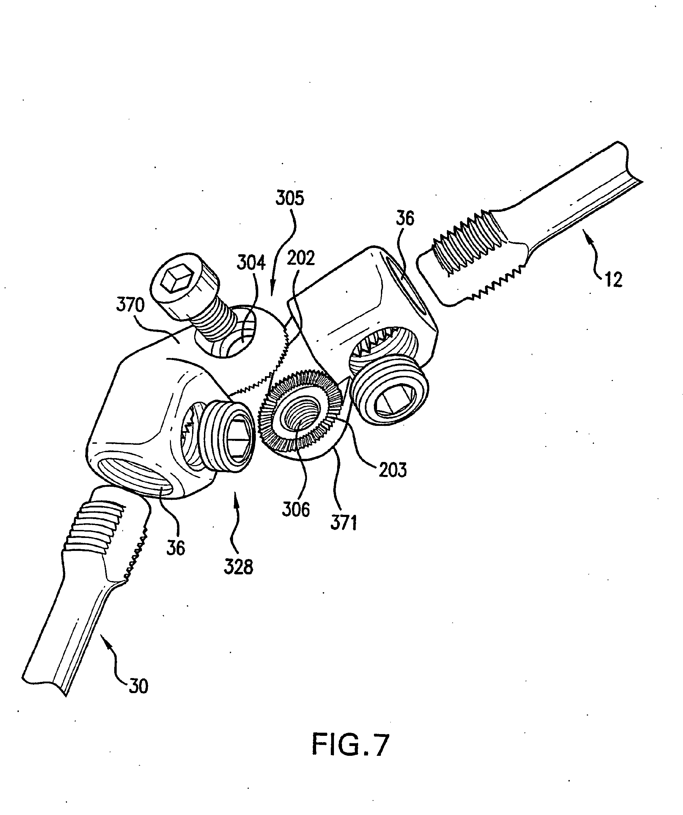

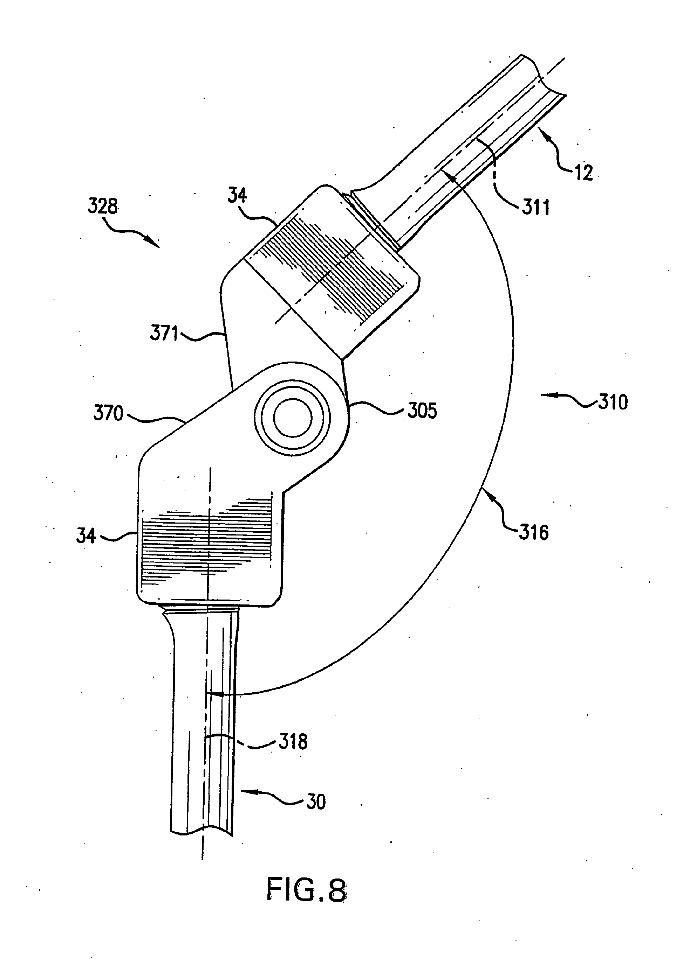

In Figs. 7-8, there is shown another embodiment of the spinal rod connection system 310. Many elements of this embodiment are identical to the elements found in the above embodiments and will not be repeated here. First rod 12 and second rod 30 are releasably coupled to one another in end-to-end fashion with a coupling mechanism 328. The coupling mechanism 328 is configured to secure rods 12, 30 to one another in end-to-end fashion while providing the necessary angulation between the axis of the rods to conform to the angle of the occiput and the sub-axial spine. In this embodiment, coupling mechanism 328 comprises an articulating connector 305 and coupling bodies 34 at either end thereof. This embodiment includes a articulating connector 305 which allows a surgeon to set and then fix the necessary angulation between the axis of the rods to conform to the angle of the occiput and the sub-axial spine while providing all of the same features and of the embodiments described above.

In Figs. 7-8, there is shown articulating connector 305 comprising extension portions 370, 371 which are respectively located at the end of coupling bodies 34 opposite of first bores 36, not shown in this figure. Extension portion 370 has a contact surface 202 that is planar and parallel to axis 318. Extension portion 371 has a contact surface 203 that is planar and parallel to axis 311. Extension portion 370 is shaped such that the axis of bore 304 is offset from axis 318. Extension portion 371 is shaped such that the axis of bore 306 is offset from axis 311. This embodiment further minimizes the footprint or intrusiveness of the coupling mechanism, and more particularly the articulating connector, into the spinal column, the base of the skull and the surrounding tissue while providing all of the same features of the embodiments described above.

While the invention has been illustrated and described in detail in the drawings and foregoing description, the same is to be considered as illustrative and not restrictive in character. All changes and modifications that come within the scope of the invention as defined by the claims are desired to be protected.

## Claims

1. A spinal rod connection (10) system, comprising:
a first elongated rod (12) for attachment to the spinal column extending along a longitudinal axis (11) between a first end and an opposite second end, said first rod (12) including a first characteristic and a first coupling member (16) at said first end;
a second elongated rod (30) for attachment to the skull extending along a longitudinal axis (8) between a first end and an opposite second end, said second rod (30) including a second characteristic and a second coupling member (16) at said first end; and
a coupling mechanism (28) for releasably coupling said first coupling member (16) and said second coupling member (16) in an end-to-end fashion, wherein said longitudinal axis (11, 8) of each of said first (12) and second (30) rods is at an angle to one another;
wherein said coupling (28) mechanism includes a first end and an opposite second end, said first end including a first coupling body (34) and said second end including a second coupling body (34) the coupling bodies (34) including each an engaging member (42);
wherein said first coupling member (16) is releasably engageable to said first coupling body (34) and said second coupling member (16) is releasably engageable to said second coupling body (34); and
wherein said first coupling body (34) includes an internally threaded first bore (36) and said first coupling member (16) is externally threaded for removable engagement in said first bore (36) and wherein said second coupling body (34) includes an internally threaded first bore (36) and said second coupling member (16) is externally threaded for removable engagement in said first bore (36); **characterised in that**
said first and second coupling members (16) include an external thread pattern interrupted by a flattened surface portion (18) extending along said coupling member (16) for contacting the engaging members.

2. The system of claim 1, wherein said coupling mechanism (28) axially and torsionally constrains said first (12) and second (30) rods relative to one another.

3. The system of claim 1, wherein said first and second characteristics each include a diameter of a respective one of first (14) and second (32) rod portions of said first (12) and second (30) rods, respectively, wherein the first diameter is greater than the second diameter.

4. The system of claim 1, wherein said first and second characteristics each include a diameter of a respective one of first (14) and second (32) rod portions of said first (12) and second (30) rods, respectively , wherein the second diameter is greater than the first diameter:

5. The system of any preceding claim wherein said flattened surface lacks threads.

6. The system of claim 5, wherein at least one of said first and second coupling bodies (34) includes a second bore (38) transversely oriented to and intersecting said first bore (36), said second bore (38) including an internal thread for engaging an externally threaded set screw (44), said set screw (44) positionable in engagement with said flattened surface (18) of said coupling member (16) when said coupling member (16) is engaged in said first bore (36) to prevent said coupling member (16) from rotating in said first bore (36).

7. The system of claim 6, wherein said second bore (38) is orthogonal to said first bore (36).

8. A spinal rod connection system (210), comprising:
a first elongated rod (12) for attachment to the spinal column extending along a longitudinal axis (211)between a first end and an opposite second end, said first rod (12) including a first characteristic and a first coupling member at said first end;
a second elongated rod (30) for attachment to the skull extending along a longitudinal axis (218) between a first end and an opposite second end, said second rod (30) including a second characteristic and a second coupling member at said first end; and
a coupling mechanism (228) for releasably coupling said first coupling member and said second coupling member in an end-to-end fashion, wherein an angle between said longitudinal axis (211, 218) of each of said first (12) and second (30) rods can be temporarily varied and thereafter fixed so that said coupling mechanism (228) axially and torsionally constrains said first (12) and second (30) rods relative to one another;
wherein said coupling mechanism (228) includes an articulating connector (205) between a first end and an opposite second end, said first end including a first coupling body (34) and said second end including a second coupling body (34) the coupling bodies (34) including each an engaging member,
wherein said first coupling member is releasably engageable to said first coupling body (34) and said second coupling member is releasably engageable to said second coupling body (34);
wherein said first coupling body (34) includes an internally threaded first bore (36) and said first coupling member is externally threaded for removable engagement in said first bore (36) and wherein said second coupling body includes an internally threaded first bore (36) and said second coupling member is externally threaded for removable engagement in said first bore (34) **characterised in that**
said first and second coupling members include an external thread pattern interrupted by a flattened surface portion expending along said coupling member for contacting the engaging members.

9. The system of claim 8, wherein said first and second characteristics each include a diameter of a respective one of first and second rod portions of said first (12) and second (30) rods, respectively, wherein the first diameter is greater than the second diameter.

10. The system of claim 8, wherein said first and second characteristics each include a diameter of a respective one of first and second rod portions of said first (12) and second (30) rods, respectively, wherein the second diameter is greater than the first diameter;

11. The system of any of claims 8 to 10, wherein said flattened surface lacks threads.

12. The system of claim 11, wherein at least one of said first and second coupling bodies (34) includes a second bore transversely oriented to and intersecting said first bore (36), said second bore including an internal thread for engaging an externally threaded set screw, said set screw positionable in engagement with said flattened surface of said coupling member when said coupling member is engaged in said first bore (36) to prevent said coupling member from rotating in said first bore (36).

13. The system of claim 12, wherein said second bore is orthogonal to said first bore (36).

14. The system of any of claims 8 to 13, wherein said articulating connector (205) comprises:
a first extension portion (271) connected to said first coupling body, said first extension portion (271) including a contact surface (203) that is parallel to the axis (211) of the first rod (12), wherein the first extension portion (271) includes a bore (206) perpendicular to the contact surface (203);
a second extension portion (270) connected to said second coupling body, said second extension portion (270) including a contact (202) surface that is parallel to the axis (218) of the second rod (30), wherein the second extension portion (270) includes an internally threaded bore (204) perpendicular to the contact surface (202);
a fastener (290) including an external threaded portion (291) for insertion through the bore (206) of said first extension portion (271) and removable engagement in the bore (204) of said second extension portion (270), whereby said first extension contact surface (203) and said second extension contact surface (202) engage one another and are prevented from articulating relative to one another.

15. The system of claim 14, wherein at least one of the contact surfaces (202, 203) include a movement prevention device.

16. The system of claim 15, wherein said movement prevention device includes splines.

## Patentansprüche

1. Wirbelsäulenstangenverbindungssystem (10), das folgendes umfasst:
eine erste elongierte Stange (12) zur Befestigung an der Wirbelsäule, die sich entlang einer Längsachse (11) zwischen einem ersten Ende und einem entgegengesetzten zweiten Ende erstreckt, wobei die genannte erste Stange (12) ein erstes Merkmal aufweist sowie ein erstes Kopplungselement (16) an dem genannten ersten Ende;
eine zweite elongierte Stange (30) zur Befestigung an dem Schädel, die sich entlang einer Längsachse (8) zwischen einem ersten Ende und einem entgegengesetzten zweiten Ende erstreckt, wobei die genannte zweite Stange (30) ein zweites Merkmal und ein zweites Kopplungselement (16) an dem genannten ersten Ende aufweist; und
einen Kopplungsmechanismus (28) zum lösbaren Koppeln des genannten ersten Kopplungselements (16) und des genannten zweiten Kopplungselement (16) Ende-an-Ende, wobei die genannte Längsachse (11, 8) die genannten ersten (12) und zweiten (30) Stangen in einem Winkel zueinander angeordnet sind;
wobei der genannte Kopplungsmechanismus (28) ein erstes Ende und ein entgegengesetztes zweites Ende aufweist, wobei das genannte erste Ende einen ersten Kopplungskörper (34) aufweist, und wobei das genannte zweite Ende einen zweiten Kopplungskörper (34) aufweist, wobei die Kopplungskörper (34) jeweils ein Eingriffselement (42) aufweisen;
wobei das genannte erste Kopplungselement (16) lösbar mit den genannten ersten Kopplungskörper (34) eingreifen kann, und wobei das genannte zweite Kopplungselement (16) lösbar mit dem genannten zweiten Kopplungskörper (34) eingreifen kann; und
wobei der genannte erste Kopplungskörper (34) eine erste Bohrung (36) mit Innengewinde aufweist, und wobei das genannte erste Kopplungselement (16) ein Außengewinde für einen lösbaren Eingriff in der genannten ersten Bohrung (36) aufweist, und wobei der genannte zweite Kopplungskörper (34) eine erste Bohrung (36) mit Innengewinde aufweist, und wobei das genannte zweite Kopplungselement (16) ein Außengewinde für einen lösbaren Eingriff in der genannten ersten Bohrung (36) aufweist; **dadurch gekennzeichnet, dass**:
die genannten ersten und zweiten Kopplungselemente (16) ein Außengewindemuster aufweisen, das durch einen abgeflachten Oberflächenabschnitt (18) unterbrochen ist, der sich entlang des genannten Kopplungselements (16) erstreckt, um Kontakt mit den genannten Eingriffselementen herzustellen.

2. System nach Anspruch 1, wobei der genannte Kopplungsmechanismus (28) axial und torsional die genannten ersten (12) und zweiten (30) Stangen im Verhältnis zueinander eingrenzt.

3. System nach Anspruch 1, wobei die genannten ersten und zweiten Merkmale jeweils einen Durchmesser eines entsprechenden ersten (14) oder zweiten (32) Stangenabschnitts der genannten ersten (12) bzw. zweiten (30) Stangen aufweisen, wobei der erste Durchmesser größer ist als der zweite Durchmesser.

4. System nach Anspruch 1, wobei die genannten ersten und zweiten Merkmale jeweils einen Durchmesser eines entsprechenden ersten (14) oder zweiten (32) Stangenabschnitts der genannten ersten (12) bzw. zweiten (30) Stangen aufweisen, wobei der zweite Durchmesser größer ist als der erste Durchmesser.

5. System nach einem der vorstehenden Ansprüche, wobei die genannte abgeflachte Oberfläche keine Gewinde aufweist.

6. System nach Anspruch 5, wobei zumindest einer der genannten ersten und zweiten Kopplungskörper (34) eine zweite Bohrung (38) aufweist, die transversal ausgerichtet ist zu der genannten ersten Bohrung (36) und diese kreuzt, wobei die genannte zweite Bohrung (38) ein Innengewinde für einen Eingriff mit einer Stellschraube (44) mit Außengewinde aufweist, wobei die genannte Stellschraube (44) in Eingriff mit der genannten abgeflachten Oberfläche (18) des genannten Kopplungselements (16) positioniert werden kann, wenn das genannte Kopplungselement (16) in der genannten ersten Bohrung (36) eingreift, um eine Rotation des genannten Kopplungselements (16) in der genannten ersten Bohrung (36) zu verhindern.

7. System nach Anspruch 6, wobei die genannte zweite Bohrung (38) orthogonal zu der genannten ersten Bohrung (36) ist.

8. Wirbelsäulenstangenverbindungssystem (210), das folgendes umfasst:
eine erste elongierte Stange (12) zur Befestigung an der Wirbelsäule, die sich entlang einer Längsachse (211) zwischen einem ersten Ende und einem entgegengesetzten zweiten Ende erstreckt, wobei die genannte erste Stange (12) ein erstes Merkmal aufweist sowie ein erstes Kopplungselement an dem genannten ersten Ende;
eine zweite elongierte Stange (30) zur Befestigung an dem Schädel, die sich entlang einer Längsachse (218) zwischen einem ersten Ende und einem entgegengesetzten zweiten Ende erstreckt, wobei die genannte zweite Stange (30) ein zweites Merkmal und ein zweites Kopplungselement an dem genannten ersten Ende aufweist; und
einen Kopplungsmechanismus (228) zum lösbaren Koppeln des genannten ersten Kopplungselements und des genannten zweiten Kopplungselement Ende-an-Ende, wobei ein Winkel zwischen den genannten Längsachsen (211, 218) jeder der genannten ersten (12) und zweiten (30) Stangen vorübergehend verändert und danach fixiert werden kann, so dass der genannte Kopplungsmechanismus (228) die genannten ersten (12) und zweiten (30) Stangen im Verhältnis zueinander axial und torsional eingrenzt;
wobei der genannte Kopplungsmechanismus (228) einen gelenkfähigen Verbinder (205) zwischen einem ersten Ende und einem entgegengesetzten zweiten Ende aufweist, wobei das genannte erste Ende einen ersten Kopplungskörper (34) aufweist, und wobei das genannte zweite Ende einen zweiten Kopplungskörper (34) aufweist, wobei die Kopplungskörper (34) jeweils ein Eingriffselement (42) aufweisen;
wobei das genannte erste Kopplungselement lösbar mit den genannten ersten Kopplungskörper (34) eingreifen kann, und wobei das genannte zweite Kopplungselement (16) lösbar mit dem genannten zweiten Kopplungskörper (34) eingreifen kann;
wobei der genannte erste Kopplungskörper (34) eine erste Bohrung (36) mit Innengewinde aufweist, und wobei das genannte erste Kopplungselement ein Außengewinde für einen lösbaren Eingriff in der genannten ersten Bohrung (36) aufweist, und wobei der genannte zweite Kopplungskörper eine erste Bohrung (36) mit Innengewinde aufweist, und wobei das genannte zweite Kopplungselement ein Außengewinde für einen lösbaren Eingriff in der genannten ersten Bohrung (34) aufweist; **dadurch gekennzeichnet, dass**:
die genannten ersten und zweiten Kopplungselemente ein Außengewindemuster aufweisen, das durch einen abgeflachten Oberflächenabschnitt unterbrochen ist, der sich entlang des genannten Kopplungselements erstreckt, um Kontakt mit den genannten Eingriffselementen herzustellen.

9. System nach Anspruch 8, wobei die genannten ersten und zweiten Merkmale jeweils einen Durchmesser eines entsprechenden ersten oder zweiten Stangenabschnitts der genannten ersten (12) bzw. zweiten (30) Stangen aufweisen, wobei der erste Durchmesser größer ist als der zweite Durchmesser.

10. System nach Anspruch 8, wobei die genannten ersten und zweiten Merkmale jeweils einen Durchmesser eines entsprechenden ersten oder zweiten Stangenabschnitts der genannten ersten (12) bzw. zweiten (30) Stangen aufweisen, wobei der zweite Durchmesser größer ist als der erste Durchmesser.

11. System nach einem der Ansprüche 8 bis 10, wobei die genannte abgeflachte Oberfläche keine Gewinde aufweist.

12. System nach Anspruch 11, wobei zumindest einer der genannten ersten und zweiten Kopplungskörper (34) eine zweite Bohrung aufweist, die transversal ausgerichtet ist zu der genannten ersten Bohrung (36) und diese kreuzt, wobei die genannte zweite Bohrung ein Innengewinde für einen Eingriff mit einer Stellschraube mit Außengewinde aufweist, wobei die genannte Stellschraube in Eingriff mit der genannten abgeflachten Oberfläche des genannten Kopplungselements positioniert werden kann, wenn das genannte Kopplungselement in der genannten ersten Bohrung (36) eingreift, um eine Rotation des genannten Kopplungselements in der genannten ersten Bohrung zu verhindern.

13. System nach Anspruch 12, wobei die genannte zweite Bohrung orthogonal zu der genannten ersten Bohrung (36) ist.

14. System nach einem der Ansprüche 8 bis 13, wobei der genannte gelenkfähige Verbinder (205) folgendes umfasst:
einen ersten Erweiterungsabschnitt (271), der mit dem genannten ersten Kopplungskörper verbunden ist, wobei der genannte erste Erweiterungsabschnitt (271) eine Kontaktoberfläche (203) aufweist, die parallel zu der Achse (211) der ersten Stange (12) ist, wobei der erste Erweiterungsabschnitt (271) eine Bohrung (206) senkrecht zu der Kontaktoberfläche (203) aufweist;
einen zweiten Erweiterungsabschnitt (270), der mit dem genannten zweiten Kopplungskörper verbunden ist, wobei der genannte zweite Erweiterungsabschnitt (270) eine Kontaktoberfläche (202) aufweist, die parallel zu der Achse (218) der zweiten Stange (30) ist, wobei der zweite Erweiterungsabschnitt (270) eine Bohrung (204) mit Innengewinde aufweist, die senkrecht zu der Kontaktoberfläche (202) ist;
eine Befestigungseinrichtung (290), die einen Außengewindeabschnitt (291) zum Einführen durch die Bohrung (206) des genannten ersten Erweiterungsabschnitts (271) sowie für einen lösbaren Eingriff in der Bohrung (204) des genannten zweiten Erweiterungsabschnitts (270) aufweist, wodurch die Kontaktoberfläche (203) des genannten ersten Erweiterungsabschnitts und die Kontaktoberfläche (202) des genannten zweiten Erweiterungsabschnitts miteinander eingreifen und es verhindert wird, dass sie sich im Verhältnis zueinander gelenkig bewegen.

15. System nach Anspruch 14, wobei mindestens eine der Kontaktoberflächen (202, 203) eine Bewegungsverhinderungsvorrichtung aufweist.

16. System nach Anspruch 15, wobei die genannte Bewegungsverhinderungsvorrichtung Keilprofile aufweist.

## Revendications

1. Système de connexion de broche spinale (10), comprenant :
une première broche allongée (12) pour une fixation à la colonne vertébrale s'étendant le long d'un axe longitudinal (11) entre une première extrémité et une seconde extrémité opposée, ladite première broche (12) comprenant une première caractéristique et un premier élément d'accouplement (16) à ladite première extrémité ;
une seconde broche allongée (30) pour une fixation sur le crâne s'étendant le long d'un axe longitudinal (8) entre une première extrémité et une seconde extrémité opposée, ladite seconde broche (30) comprenant une seconde caractéristique et un second élément d'accouplement (16) au niveau de ladite première extrémité ; et
un mécanisme d'accouplement (28) pour accoupler de manière libérable ledit premier élément d'accouplement (16) et ledit second élément d'accouplement (16) d'une manière bout à bout, dans lequel ledit axe longitudinal (11, 8) de chacune desdites première (12) et seconde (30) broches est à un angle par rapport à l'autre ;
dans lequel ledit (28) mécanisme d'accouplement comprend une première extrémité et une seconde extrémité opposée, ladite première extrémité comprenant un premier corps d'accouplement (34) et ladite seconde extrémité comprenant un second corps d'accouplement (34), les corps d'accouplement (34) comprenant chacun un élément de mise en prise (42) ;
dans lequel ledit premier élément d'accouplement (16) est en prise de façon libérable avec ledit premier corps d'accouplement (34) et ledit second élément d'accouplement (16) est en prise de manière libérable avec ledit second corps d'accouplement (34) ; et
dans lequel ledit premier corps d'accouplement (34) comprend un premier alésage fileté intérieurement (36) et ledit premier élément d'accouplement (16) est fileté extérieurement pour une prise amovible dans ledit premier alésage (36) et dans lequel ledit second corps d'accouplement (34) comprend un premier alésage fileté intérieurement (36) et ledit second élément d'accouplement (16) est fileté extérieurement pour venir en prise amovible dans ledit premier alésage (36), **caractérisé en ce que**
lesdits premier et second éléments d'accouplement (16) comprennent un motif de filetage externe interrompu par une partie de surface aplatie (18) s'étendant le long dudit élément d'accouplement (16) pour mettre en contact les éléments de mise en prise.

2. Système selon la revendication 1, dans lequel ledit mécanisme d'accouplement (28) contraint axialement et en torsion lesdites première (12) et seconde broches (30) l'une par rapport à l'autre.

3. Système selon la revendication 1, dans lequel lesdites première et seconde caractéristiques comprennent chacune un diamètre de l'une respective des première (14) et seconde (32) parties de broche desdites première (12) et seconde (30) broches, respectivement, dans lequel le premier diamètre est plus grand que le second diamètre.

4. Système selon la revendication 1, dans lequel lesdites première et seconde caractéristiques comprennent chacune un diamètre de l'une respective des première (14) et seconde (32) parties de broche desdites première (12) et seconde (30) broches, respectivement, dans lequel le second diamètre est plus grand que le premier diamètre.

5. Système selon l'une quelconque des revendications précédentes, dans lequel ladite surface aplatie n'est pas filetée.

6. Système selon la revendication 5, dans lequel au moins l'un desdits premier et second corps d'accouplement (34) comprend un second alésage (38) orienté transversalement à et coupant ledit premier alésage (36), ledit second alésage (38) comprenant un filetage interne pour venir en prise avec une vis de réglage à filetage externe (44), ladite vis de réglage (44) pouvant être positionnée en prise avec ladite surface aplatie (18) dudit élément d'accouplement (16) lorsque ledit élément d'accouplement (16) est en prise dans ledit premier alésage (36) pour empêcher ledit élément d'accouplement (16) de tourner dans ledit premier alésage (36).

7. Système selon la revendication 6, dans lequel ledit second alésage (38) est orthogonal audit premier alésage (36).

8. Système de connexion de broche spinale (210), comprenant :
une première broche allongée (12) pour une fixation à la colonne vertébrale s'étendant le long d'un axe longitudinal (211) entre une première extrémité et une seconde extrémité opposée, ladite première broche (12) comprenant une première caractéristique et un premier élément d'accouplement à ladite première extrémité ;
une seconde broche allongée (30) pour une fixation sur le crâne s'étendant le long d'un axe longitudinal (218) entre une première extrémité et une seconde extrémité opposée, ladite seconde broche (30) comprenant une seconde caractéristique et un second élément d'accouplement au niveau de ladite première extrémité ; et
un mécanisme d'accouplement (228) pour coupler de manière libérable ledit premier élément d'accouplement et ledit second élément d'accouplement d'une manière bout à bout, dans lequel un angle entre ledit axe longitudinal (211, 218) de chacune desdites première (12) et seconde (30) broches peut être temporairement modifié et ensuite fixé de manière que ledit mécanisme d'accouplement (228) contraint axialement et en torsion lesdites première (12) et seconde (30) broches l'une par rapport à l'autre ;
dans lequel ledit mécanisme d'accouplement (228) comprend un connecteur d'articulation (205) entre une première extrémité et une seconde extrémité opposée, ladite première extrémité comprenant un premier corps d'accouplement (34) et ladite seconde extrémité comprenant un second corps d'accouplement (34), les corps d'accouplement (34) comprenant chacun un élément de mise en prise (42) ;
dans lequel ledit premier élément d'accouplement est en prise de façon libérable avec ledit premier corps d'accouplement (34) et ledit second élément d'accouplement est en prise de manière libérable avec ledit second corps d'accouplement (34) ;
dans lequel ledit premier corps d'accouplement (34) comprend un premier alésage fileté intérieurement (36) et ledit premier élément d'accouplement est fileté extérieurement pour une prise amovible dans ledit premier alésage (36) et dans lequel ledit second corps d'accouplement comprend un premier alésage fileté intérieurement (36) et ledit second élément d'accouplement est fileté extérieurement pour venir en prise amovible dans ledit premier alésage (34), **caractérisé en ce que**
lesdits premier et second éléments d'accouplement comprennent un motif de filetage externe interrompu par une partie de surface aplatie s'étendant le long dudit élément d'accouplement pour mettre en contact les éléments de mise en prise.

9. Système selon la revendication 8, dans lequel lesdites première et seconde caractéristiques comprennent chacune un diamètre de l'une respective des première et seconde parties de broche desdites première (12) et seconde (30) broches, respectivement, dans lequel le premier diamètre est plus grand que le second diamètre.

10. Système selon la revendication 8, dans lequel lesdites première et seconde caractéristiques comprennent chacune un diamètre de l'une respective des première et seconde parties de broche desdites première (12) et seconde (30) broches, respectivement, dans lequel le second diamètre est plus grand que le premier diamètre.

11. Système selon l'une quelconque des revendications 8 à 10, dans lequel ladite surface aplatie n'est pas filetée.

12. Système selon la revendication 11, dans lequel au moins l'un desdits premier et second corps d'accouplement (34) comprend un second alésage orienté transversalement à et coupant ledit premier alésage (36), ledit second alésage comprenant un filetage interne pour venir en prise avec une vis de réglage à filetage externe, ladite vis de réglage pouvant être positionnée en prise avec ladite surface aplatie dudit élément d'accouplement lorsque ledit élément d'accouplement est en prise dans ledit premier alésage (36) pour empêcher ledit élément d'accouplement de tourner dans ledit premier alésage (36).

13. Système selon la revendication 12, dans lequel ledit second alésage est orthogonal audit premier alésage (36).

14. Système selon l'une quelconque des revendications 8 à 13, dans lequel ledit connecteur d'articulation (205) comprend :
une première partie d'extension (271) reliée audit premier corps d'accouplement, ladite première partie d'extension (271) comprenant une surface de contact (203) qui est parallèle à l'axe (211) de la première broche (12), dans lequel la première partie d'extension (271) comprend un alésage (206) perpendiculaire à la surface de contact (203) ;
une seconde partie d'extension (270) reliée audit second corps d'accouplement, ladite seconde partie d'extension (270) comprenant une surface de contact (202) qui est parallèle à l'axe (218) de la seconde broche (30), dans lequel la seconde partie d'extension (270) comprend un alésage fileté intérieurement (204) perpendiculaire à la surface de contact (202) ;
un élément de fixation (290) comprenant une partie filetée externe (291) pour insertion à travers l'alésage (206) de ladite première partie d'extension (271) et une prise libérable dans l'alésage (204) de ladite seconde partie d'extension (270), moyennant quoi ladite première surface de contact d'extension (203) et ladite seconde surface de contact d'extension (202) viennent en prise l'une avec l'autre et sont empêchées de s'articuler l'une par rapport à l'autre.

15. Système selon la revendication 14, dans lequel au moins l'une des surfaces de contact (202, 203) comprend un dispositif de prévention de mouvement.

16. Système selon la revendication 15, dans lequel ledit dispositif de prévention de mouvement comprend des cannelures.
